# EUROPEAN PATENT APPLICATION

(11) **EP 1 982 991 A1**
(43) Date of publication of application: **22.10.2008**
(21) Application number: 07713921.0
(22) Date of filing: 08.02.2007
(51) Int. Cl.: C07K 1/06, G01N 33/68

(54) **METHOD FOR MODIFYING PEPTIDE AND METHOD FOR IDENTIFYING PEPTIDE**

(30) Priority: 08.02.2006 JP 2006031098
(71) Applicant: NEC Corporation, Tokyo 108-0014 (JP)
(72) Inventor: MIYAZAKI, Kenji, Tokyo 108-0014 (JP); TABUSE, Yo, Tokyo 108-0014 (JP); MINAGAWA, Hirotaka, Tokyo 108-0014 (JP); SAITO, Emiko, Tokyo 108-0014 (JP); KAMIJO, Ken'ichi, Tokyo 108-0014 (JP); TSUGITA, Akira, Tokyo 108-0014 (JP)
(74) Representative: Betten & Resch
(86) International application number: PCT/JP2007/052205
(87) International publication number: WO 2007/091628

(57) **Abstract**

**DISCLOSURE OF THE INVENTION**

The present invention is to provide a method for easily and specifically modifying specific amino acid residue(s) constituting a peptides and to provide a methodology of improving the accuracy of identification of the peptide using a new information of the peptide obtained from the number of modified amino acid residue by said specific modification method as mentioned. The method for modifying a peptide according to the present invention is **characterized**:
A method for modifying a peptide, wherein the peptide as supported in a substrate and an aqueous solution of perhalogenated carboxylic acid containing an alcohol is reacted to selectively esterify a glutamic acid residue of said peptide.
The method for identifying a peptide according to the present invention is **characterized**:
A method for identifying a peptide comprising the steps of:
reacting the peptide as supported in a substrate and an aqueous solution of perhalogenated carboxylic acid containing an alcohol to selectively esterify glutamic acid residue of said peptide;
immersing said substrate in a protease solution to obtain a peptide fragment originated from said peptide;
measuring a molecular weight of said peptide fragment; and
determining said peptide based on said molecular weight.

SEQUENCE LISTING

<110>  NEC Corporation

<120>  A method for modifying a peptide and a method for
identifying a peptide

<130>  341-04023

<150>  JP2006-031098
<151>  2006-02-08

<160>  9

<170>  PatentIn version 3.1

<210>  1
<211>  153
<212>  PRT
<213>  Equus caballus

<400>  1

Gly Leu Ser Asp Gly Glu Trp Gln Gln Val Leu Asn Val Trp Gly Lys
1               5                   10                  15

Val Glu Ala Asp Ile Ala Gly His Gly Gln Glu Val Leu Ile Arg Leu
            20                  25                  30

Phe Thr Gly His Pro Glu Thr Leu Glu Lys Phe Asp Lys Phe Lys His
        35                  40                  45

Leu Lys Thr Glu Ala Glu Met Lys Ala Ser Glu Asp Leu Lys Lys His
    50                  55                  60

Gly Thr Val Val Leu Thr Ala Leu Gly Gly Ile Leu Lys Lys Lys Gly
65                  70                  75                  80

His His Glu Ala Glu Leu Lys Pro Leu Ala Gln Ser His Ala Thr Lys
                85                  90                  95

His Lys Ile Pro Ile Lys Tyr Leu Glu Phe Ile Ser Asp Ala Ile Ile
            100                 105                 110

His Val Leu His Ser Lys His Pro Gly Asp Phe Gly Ala Asp Ala Gln
        115                 120                 125

Gly Ala Met Thr Lys Ala Leu Glu Leu Phe Arg Asn Asp Ile Ala Ala
    130                 135                 140

Lys Tyr Lys Glu Leu Gly Phe Gln Gly
145                 150

<210>  2
<211>  6
<212>  PRT
<213>  Equus caballus

<400>  2

Ala Leu Glu Leu Phe Arg

1               5

<210>  3
<211>  11
<212>  PRT
<213>  Equus caballus

<400>  3

Leu Phe Thr Gly His Pro Glu Thr Leu Glu Lys
1               5                   10

<210>  4
<211>  14
<212>  PRT
<213>  Equus caballus

<400>  4

His Gly Thr Val Val Leu Thr Ala Leu Gly Gly Ile Leu Lys
1               5                   10

<210>  5
<211>  15
<212>  PRT
<213>  Equus caballus

<400>  5

His Pro Gly Asp Phe Gly Ala Asp Ala Gln Gly Ala Met Thr Lys
1               5                   10                  15

<210> 6
<211> 15
<212> PRT
<213> Equus caballus

<400> 6

Val Glu Ala Asp Ile Ala Gly His Gly Gln Glu Val Leu Ile Arg
1               5                   10                  15

<210> 7
<211> 16
<212> PRT
<213> Equus caballus

<400> 7

Gly Leu Ser Asp Gly Glu Trp Gln Gln Val Leu Asn Val Trp Gly Lys
1               5                   10                  15

<210> 8
<211> 17
<212> PRT
<213> Equus caballus

<400> 8

Gly His His Glu Ala Glu Leu Lys Pro Leu Ala Gln Ser His Ala Thr
1               5                   10                  15

Lys

<210>  9

<211>  16

<212>  PRT

<213>  Equus caballus

<400>  9

Tyr Leu Glu Phe Ile Ser Asp Ala Ile Ile His Val Leu His Ser Lys
1               5                   10                  15

SEQUENCE LISTING

<110> NEC Corporation

<120> A method for modifying a peptide and a method for
identifying a peptide

<130> 341-04023

<150> JP2006-031098
<151> 2006-02-08

<160> 9

<170> PatentIn version 3.1

<210> 1
<211> 153
<212> PRT
<213> Equus caballus

<400> 1

Gly Leu Ser Asp Gly Glu Trp Gln Gln Val Leu Asn Val Trp Gly Lys
1               5                   10                  15

Val Glu Ala Asp Ile Ala Gly His Gly Gln Glu Val Leu Ile Arg Leu
            20                  25                  30

Phe Thr Gly His Pro Glu Thr Leu Glu Lys Phe Asp Lys Phe Lys His
        35                  40                  45

Leu Lys Thr Glu Ala Glu Met Lys Ala Ser Glu Asp Leu Lys Lys His
    50                  55                  60

Gly Thr Val Val Leu Thr Ala Leu Gly Gly Ile Leu Lys Lys Lys Gly
65                  70                  75                  80

His His Glu Ala Glu Leu Lys Pro Leu Ala Gln Ser His Ala Thr Lys
                85                  90                  95

His Lys Ile Pro Ile Lys Tyr Leu Glu Phe Ile Ser Asp Ala Ile Ile
            100                 105                 110

His Val Leu His Ser Lys His Pro Gly Asp Phe Gly Ala Asp Ala Gln
        115                 120                 125

Gly Ala Met Thr Lys Ala Leu Glu Leu Phe Arg Asn Asp Ile Ala Ala
    130                 135                 140

Lys Tyr Lys Glu Leu Gly Phe Gln Gly
145                 150

<210> 2
<211> 6
<212> PRT
<213> Equus caballus

<400> 2

Ala Leu Glu Leu Phe Arg

1               5

<210>  3
<211>  11
<212>  PRT
<213>  Equus caballus

<400>  3

Leu Phe Thr Gly His Pro Glu Thr Leu Glu Lys
1               5                   10

<210>  4
<211>  14
<212>  PRT
<213>  Equus caballus

<400>  4

His Gly Thr Val Val Leu Thr Ala Leu Gly Gly Ile Leu Lys
1               5                   10

<210>  5
<211>  15
<212>  PRT
<213>  Equus caballus

<400>  5

His Pro Gly Asp Phe Gly Ala Asp Ala Gln Gly Ala Met Thr Lys
1               5                   10                  15

<210> 6

<211> 15

<212> PRT

<213> Equus caballus

<400> 6

Val Glu Ala Asp Ile Ala Gly His Gly Gln Glu Val Leu Ile Arg
1               5                   10                  15

<210> 7

<211> 16

<212> PRT

<213> Equus caballus

<400> 7

Gly Leu Ser Asp Gly Glu Trp Gln Gln Val Leu Asn Val Trp Gly Lys
1               5                   10                  15

<210> 8

<211> 17

<212> PRT

<213> Equus caballus

<400> 8

Gly His His Glu Ala Glu Leu Lys Pro Leu Ala Gln Ser His Ala Thr
1               5                   10                  15

Lys

<210>  9

<211>  16

<212>  PRT

<213>  Equus caballus

<400>  9

Tyr Leu Glu Phe Ile Ser Asp Ala Ile Ile His Val Leu His Ser Lys
1               5                   10                  15

## Description

### FIELD OF THE INVENTION

The present invention relates to a method for modifying a peptide and a method for identifying a peptide using the same.

### BACKGROUD OF THE INVENTION

Information of amino acid sequences is indispensable for researching the biological characterization and function of natural peptides and proteins (hereinafter, correctively referred also to as "peptide"). At present, amino acid sequences such as that of the peptides and the proteins is determined as a deduced amino acid sequence based on the corresponding gene information, that is, base sequence of genome gene encoding these amino acid sequences and of cDNA prepared from mRNA. In addition, these amino acid sequences of the protein are determined from any methods for directly analyzing the sequence of the peptide. In such cases, the information of the amino acid sequence of the peptide is still necessary for specifying genome gene encoding the peptide and cDNA prepared from mRNA.

Method for obtaining information of an amino acid sequence includes the peptide mass fingerprint (PMF) method. This method is a method for identifying the original peptide based on information such as molecular weight in which the separated band as electrophored, for example, in SDS-PAGE is cut out, the band is digested into a plurality of peptide fragments using proteolytic enzyme(s) such as cutting-site-specific protease, and information of each peptide fragment such as the molecular weight is measured in accordance with mass analysis. In order to identifying the peptide using this method with enough accuracy, it is necessary to generate at least 4 to 5 peptide fragments by digesting the peptide.

However, in the case of a peptide having low molecular weight to be measured, it is often the case that number of peptide fragment obtained from the treatment of the proteolytic enzyme is limited. In addition, even in the case of a peptide having high molecular weight to be measured, it is considered that enough number of peptides fragments cannot be obtained, since the number of peptide fragment as obtained by digesting the peptide with proteolytic enzyme depends on the manner of the proteolytic enzyme.

Method for improving the accuracy of the identification of the peptide using PMF method includes a method for comparing the spectrum of mass analysis of the peptide fragment obtained, from the digestion with several proteolytic enzymes. However, in this method, many spectrums will be obtained, and peaks to be compared are obtained, whereby making the identification complicate.

In addition, the another method includes a method for comparing the molecular weights of the pre-modified and post-modified peptide fragment originated from the peptide, for example by unspecifically modifying amino acid residue consisted of the peptide. However, this method has a problem that the step of tracing/comparing a change between the pre-modified fragment and the post-modified fragment is complex, since the peptide is unspecifically modified.

Further, the still another method may include a method for comparing molecular weights of pre-modified and post-modified peptide fragment originated from the peptide using enzyme specifically modifying the amino acid residue of the peptide. However, it is difficult to react such an enzyme in the gel. In addition, this method has problems that in the step of obtaining the molecular weight information after the modification reaction, enzyme fragment originated from the modification enzyme which is subjected to the cleavage treatment of the proteolytic enzyme affects the peak of the peptide fragment to be targeted. So, this method is not convenient.

Further, even in the case that enough number of peptide fragments will be theoretically obtained, some of the obtained peptide fragments may not be observed in the molecular weight measurement using mass analysis because of multiple action including considerable heterogeneity of ionization tendency and the suppression effect. Even in any cases, there is a need for improving the accuracy of identifying the peptides using PMF method.

It should be noted that the present applicant cannot find any prior art published documents related to the present invention before the filling of the present application. Therefore, information of prior art document is not disclosed.

### SUMMARY OF THE DISCLOSURE

### Problem to be solved in the Invention

The present invention is made based on the above-mentioned problem, and is to provide method for easily and specifically modifying specific amino acid residue(s) constituting a peptide. In addition, the present invention is to provide methodology of improving the accuracy of identification of the peptide using a new information of the peptide obtained from the number of modified amino acid residue by said specific modification method as mentioned.

### Means for solving the problem

The method for modifying a peptide according to the present invention is characterized:
A method for modifying a peptide, wherein the peptide as supported in a substrate and an aqueous solution of perhalogenated carboxylic acid containing an alcohol is reacted to selectively esterify a glutamic acid residue of said peptide.

In addition, the method for identifying a peptide according to the present invention is characterized:
A method for identifying a peptide comprising the steps of:
   reacting the peptide as supported in a substrate and an aqueous solution of perhalogenated carboxylic acid containing an alcohol to selectively esterify glutamic acid residue of said peptide;
   immersing said substrate in a protease solution to obtain a peptide fragment originated from said peptide;
   measuring a molecular weight of said peptide fragment; and
   determining said peptide based on said molecular weight.

### Effect of the Invention

According to the present invention, it is possible to specifically modifying the glutamic acid residue of the peptide in the substrate. In addition, the identification accuracy of the peptide is improved, since the number of glutamic acid(s) constituting the peptide as a result of this modification method.

### BRIEF DESCRIPTION OF THE DRAWING

Figure 1 shows sequence of horse myoglobin, deduced sequence of digested fragment as digested with trypsin and observed sequence of digested fragment.
Figure 2 is figures showing spectrum of mass analysis of myoglobin as obtained in accordance with the method of the present invention in which the glutamic acid residue thereof is esterified, wherein (A) corresponds to the trypsin digestion of horse myoglobin, and (B) corresponds to the trypsin digestion of horse myoglobin which glutamic acid residues is esterified.
Figure 3 is a spectrum of mass analysis of the 134 to 139 residues of the myoglobin (Fragment a), wherein upper spectrum corresponds not to perform the reacting step of the present invention, and lower spectrum corresponds to perform the reacting step of the present invention.
Figure 4 is a spectrum of mass analysis of the 32 to 42 residues of the myoglobin (Fragment b), wherein upper spectrum corresponds not to perform the reacting step of the present invention, and lower spectrum corresponds to perform the reacting step of the present invention.
Figure 5 is a spectrum of mass analysis of the 64 to 77 residues of the myoglobin (Fragment c), wherein upper spectrum corresponds not to perform the reacting step of the present invention, and lower spectrum corresponds to perform the reacting step of the present invention.
Figure 6 is a spectrum of mass analysis of the 119 to 133 residues of the myoglobin (Fragment d), wherein upper spectrum corresponds not to perform the reacting step of the present invention, and lower spectrum corresponds to perform the reacting step of the present invention.
Figure 7 is a spectrum of mass analysis of the 17 to 31 residues of the myoglobin (Fragment e), wherein upper spectrum corresponds not to perform the reacting step of the present invention, and lower spectrum corresponds to perform the reacting step of the present invention.
Figure is a spectrum of mass analysis of the 17 to 31 residues (Fragment f) and 80 to 96 (Fragment g) of the myoglobin, wherein upper spectrum corresponds not to perform the reacting step of the present invention, and lower spectrum corresponds to perform the reacting step of the present invention.
Figure 9 is a spectrum of mass analysis of the 103 to 118 residues of the myoglobin (Fragment h), wherein upper spectrum corresponds not to perform the reacting step of the present invention, and lower spectrum corresponds to perform the reacting step of the present invention.

### BEST MODE FOR CARRYING OUT THE INVENTION

### (The method for modifying a peptide according to the present invention)

The method for modifying a peptide according to the present invention is characterized:
A method for modifying a peptide, wherein the peptide as supported in a substrate and an aqueous solution of perhalogenated carboxylic acid containing an alcohol is reacted to selectively esterify a glutamic acid residue of said peptide.

In the present invention, the substrate is not limited so far as it is a gel composition capable of retaining the peptide, and includes, for example, polyacrylamide gel. The substrate can be a member used not only for conventional one dimensional SDS-PAGE, but also for two-dimensional electrophoresis, as gel electrophoresis.

In the present invention, the alcohol is not limited so far as it is a compound containing an alcoholic hydroxyl group capable of esterification reaction with the amino acid residue, and can be, for example, alcohol having 1 to 5 carbon atoms. Examples of the alcohol include lower alcohols such as methanol, ethanol, propanol and butanol. Among them, methanol is preferable. In addition, atomic group of the alcohol other than the hydroxyl group can be one having various masses, and includes, for example, in the case of hydrogen, ²H and group having the same. These atoms is not limited so far as it does not inhibit the esterification reaction of alcohol with the amino acid residue, as mentioned above.

In the present invention, the peptide may or may not contain the glutamic acid residue. The peptide may be a peptide which comprises intramolecular bonding such as S-S bonding, is coordinated with metal, changes the states of the side chain in accordance with the redox statues, and can be subjected to post-translational modification such as phosphate and sugar chain. In the case that S-S bonding sterically inhibits the attack of the reagent to the glutamic acid, the present invention is applicable by reducing reaction to cleavage the S-S bonding according to the conventional method. In addition, molecular weight of the peptide is not limited so far as it can be retained in the substrate, and may be, being not limited to, in the range of 7 to 200 KDa.

In the present invention, the perhalogenated carboxylic acid is not limited so far as it contains lower carbon chain and also contains any halogen atoms such as fluorine and chlorine. For example, it includes trichloroacetic acid, trifluoroacetic acid, pentafluoropropionic acid and heptafluorobutyric acid. Among them, trifluoroacetic acid is preferable, in view of permeability to the substrate.

In the method for modifying a peptide according to the present invention, reacting condition such as temperature, time, concentration and pH may be appropriately adjusted.

The reacting temperature is not limited to far as the peptide is not eluted from the substrate and the liquid phase can be held in the liquid state. The temperature may be preferably in the range of 0 to 40°C, more preferably in the range of 20 to 40°C, and especially preferably in the range of room temperature to 25°C. In the case of exceeding 40°C, side reaction such as the cleavage reaction of the peptide with the perhalogenated carboxylic acid will be occurred. In addition, in the case of less than 0°C, the reaction will not be in progress, since the liquid phase is solidified.

The reacting time may be changed in accordance with the reacting temperature, and be, for example, in the range of 2 to 24 hours. In the case of less than 2 hours, the selective esterification reaction of the glutamic acid residue with the perhalogenated carboxylic acid is not enough in progress. In the case of exceeding 24 hours, side reaction such as esterification of aspartic residue of the peptide is occurred. In addition, in the case of reacting in the long duration, several problems is occurred that the signal(s) of the spectrum of mass analysis of the peptide as obtained from the method for modifying a peptide according to the present invention are distorted, side reaction such as hydrolysis of amino acids of serine and threonine is occurred, and the sample peptide is eluted from the substrate.

Concentrations of each component of the aqueous solution of the perhalogenated carboxylic acid containing the alcohol used for the method for modifying a peptide according to the present invention is not limited in that the concentration of the alcohol is preferable in the range of 5 to 30 v/v%, and the concentration of the perhalogenated carboxylic acid is preferable in the range of 10 to 40 v/v%. In the case of using methanol as the alcohol and less than 1 v/v% of the methanol concentration, the alcohol supplied to the glutamic acid is consumed for the reaction with the acid, thereby lacking the alcohol. In the case of exceeding 60 v/v%, the substrate is dehydrated and contracted, thereby preventing the progress of the reaction. In the case of less than 1 v/v% of the perhalogenated carboxylic acid concentration, the acid is consumed for the esterification reaction with the alcohol, thereby lacking the catalyst. In the case of exceeding 60 v/v%, good result is not obtained, since unnecessary dehydration is in progress. Among the concentrations of each component, in the case of using methanol as the alcohol and trifluoroacetic acid as the perhalogenated carboxylic acid, with regard to the concentration of the aqueous solution of the perhalogenated carboxylic acid containing the alcohol is preferably 100 to 600: 150 to 350: 300 to 700 of the volume ratio of water, methanol and trifluoroacetic acid, and more preferably 500:250:500, in view of obtainable good results.

According to the method for modifying a peptide of the present invention, carboxylic group of the glutamic acid residue of the peptide supported in the substrate is only specifically transformed, into the corresponding esterified alcohol (e.g. methylcarboxyl group, in the case of methanol).

### (The method for identifying a peptide according to the present invention)

The method for identifying a peptide according to the present invention is characterized:
A method for identifying a peptide comprising the steps of:
   reacting the peptide as supported in a substrate and an aqueous solution of perhalogenated carboxylic acid containing an alcohol to selectively esterify glutamic acid residue of said peptides;
   immersing said substrate in a protease solution to obtain a peptide fragment originated from said peptide;
   measuring a molecular weight of said peptide fragment; and
   determining said peptide based on said molecular weight. Hereinafter, each step is referred to as a reacting step, a digesting step, a measuring step and a determining step.

### (The reacting step)

The reacting step is based on the above-mentioned method for modifying a peptide according to the present invention. Thereby, the glutamic acid residue of the peptide supported in the substrate is transformed into the corresponding ester (e.g. in the case of using methanol, its methylated molecule) of the alcohol used in the reaction. The material, condition and the others of the reaction are mentioned above. It should be noted that a substituent group, corresponding to the alcohols, bound to the glutamic acid residue of the peptide as esterified by the reacting step is referred to as "substituted residue".

After the reaction is made using the peptide, the alcohol and the perhalogenated carboxylic acid with the above-mentioned predetermined condition, pH of the substrate/liquid phase may be adjusted with any pH regulators in the reacting step. In such case, the preferred pH is as the optimum pH of the protease as used in the following digesting step, and may be preferably in the range of 7 to 9. In addition, the conventional pH regulators can be preferably used as the pH regulator. For example, the preferred pH regulator includes ammonium bicarbonate solution.

Further, a dewatering treatment for removing water in the gel can be performed in the reacting step. Materials used for the dewatering treatment is not limited so far as the gel material is not solubilized and the material has an affinity for water, and may be, for example, a poplar aprotic solvent including nitriles having 4 or less of carbon atoms such as acetonitrile (CH₃CN), and ketones having 4 or less of carbon atoms such as acetone. According to the dewatering treatment, water solvent maintaining the pore size of the porous structure of the substrate is removed, and the subject peptide can be maintained in a state of being retained in the substrate.

The dewatering treatment can be performed in dependant on the desired degree of dewatering. For example, the piece of the substrate may be immersed in the dehydrating agent with several times. In addition, the piece of the substrate may be immersed for 20 minutes with three times. In the case that the piece of the substrate is stained with any dyes such as CBB (coomassie brilliant blue), the dye is removed due to solvent replacement, and the piece of the substrate will be decolored. Therefore, it can be recognized that the solvent replacement is finished by the change of the color.

In addition, in the present invention, the intramolecular/intermolecular bonding, or molecular group(s) of post-translational modification may be subjected to a degradation/cleavage treatment using an appropriate agent, prior to the reacting step. For example, in the case of the peptide containing S-S bonding in the intermolecule or intramolecule to be subjected, the S-S bonding may be cleaved to reduce it with reducing agents such as DTT. In addition, so obtained SH group as reduced from the S-S bonding may be further treated with alkylating agent to prevent the re-formation of S-S bonding.

### (The digesting step)

The digesting step is a step of immersing the substrate in a protease solution. The protease is not limited so far as it can cleave the subject peptide at the predetermined position. For example, trypsin, one of serine proteases as well known in the art, may be used. Thereby, the peptide as supported in the substrate is cleaved into the peptide fragment as cleaved at the predetermined position with the action of the protease.

It should be noted that the peptide fragment originated from the peptide as supported in the substrate is eluted from the substrate into the liquid phase with the digesting step, and the liquid phase is supplied for the following measuring step.

### (The measuring step)

The measuring step is a step of measuring a molecular weight of the peptide and/or the peptide fragment. The measuring step is not limited so far as it can measure the molecular weight of the peptide, and may be performed with various mass spectroscopes. Examples of the mass spectroscope includes ion trap mass spectrometer, quadrupole mass spectrometer, magnetic sector-type mass spectrometer, time-of-flight mass spectrometer and fourier transform mass spectrometer. In addition, examples of the ionization method includes electrospray ionization (ESI) method, matrix-assisted laser desorption/ionization (MALDI) method and fast atom bombardment (FAB) method.

Among them, MALDI-TOF-MS is preferably used. Thereby, MALDI-TOF-MS can suppress the lack of a part of the atomic group from the amino acid residue constituting the peptide fragment in the ionization process. In addition, it is possible to preferably measure the molecular weight of the peptide fragment having relatively high molecular weight. In the case that the subject proteins is separated from the sample to electrophore in the gel, that the gel is subjected to the above-mentioned treatment, and that it is recovered to supply the measurement, it is possible to measure both of the corresponding anion and cation. Therefore, an improved and reproducible analysis can be performed with MALDI-TOF-MS.

In the case of using MALDI-TOF-MS in the mass analysis, it is possible to measure both of the cationic species as added proton (H⁺) to the peptide fragment and the anionic species as leaved proton from the peptide fragment.

### (The determining step)

The determining step is a step of determining the peptide based on information such as the molecular weight of the peptide fragment originated from the peptide as obtained in the above-mentioned procedure.

An approach of assigning a peptide on the database based on the molecular weight of the peptide fragment originated from the peptide as obtained from the mass analysis has been publicly known in the PMF method. That is, the spectrum of mass analysis for the peptide fragment obtained from the combination of a specific peptide and a specific protease is unambiguous, because of the cutting-site specificity of the protease. In the method for identifying a peptide According to the present invention, the determining step can be performed with such an approach welt known in the art.

The determining step can be performed based on the molecular weights of the peptide fragments corresponding to the group as performed the above-mentioned reacting step and as not performed the reacting step of the peptide. In the case of containing the glutamic acid residue in the peptide, the substituted residue is bound to the glutamic acid residue with the determining step. The substituted residue as bound will reflect on the spectrum of mass analysis as the change of the molecular weight. Therefore, in the case of focusing on a specific peptide fragment, the change of the molecular weight originated from the substituted residue originated from performing or not performing the determining step will reflect that at least one glutamic acid residue is contained in this specific peptide fragment.

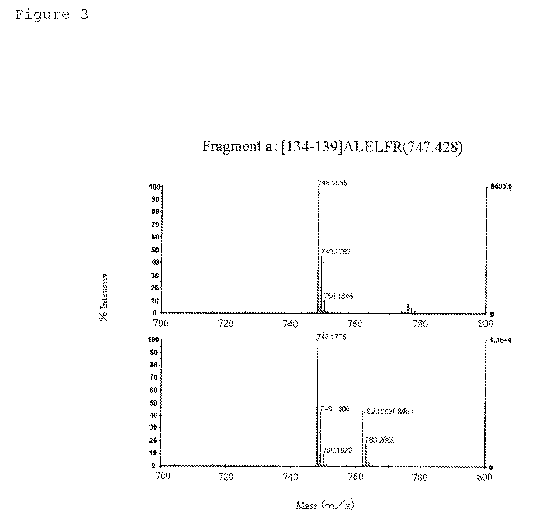

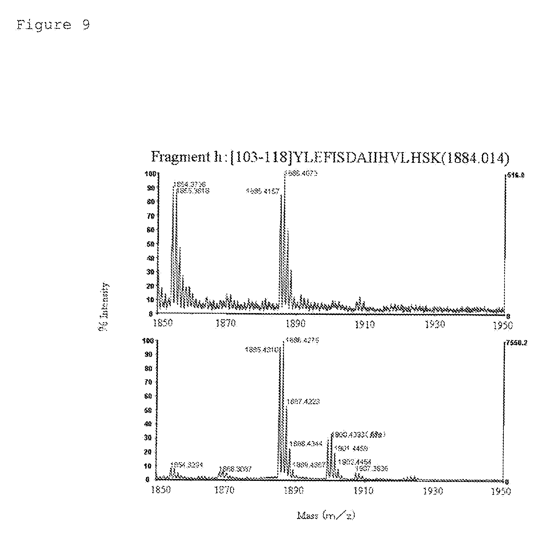

This is exemplarily shown in Figure 3. Figure 3 is a spectrum of mass analysis of the 134 to 139 residues of the myoglobin (Fragment a), wherein upper spectrum corresponds not to perform the reacting step of the present invention, and lower spectrum corresponds to perform the reacting step of the present invention. According to a comparison between the upper spectrum and lower spectrum, a peak around 762 (m/z) which does not appear in the upper spectrum is appeared in the lower spectrum. This indicates that the glutamic acid residue is contained in the peptide fragment of 134 to 139 residues of the myoglobin in relation to the molecular weight of the Fragment a (747.428) as observed in the upper spectrum. It should be noted that, as shown in the Sequence Number 3, one glutamic acid residue is contained in the sequence of the peptide fragment of 134 to 139 residues of the myoglobin, clearly supporting the result of Figure 3. In a similar way, as shown in Figure 9, the sequence of the peptide fragment of 103 to 118 residues of the myoglobin contains the glutamic acid residue, and this is shown in the peak around 1900 as observed in comparison with upper spectrum and lower spectrum of Figure 9.

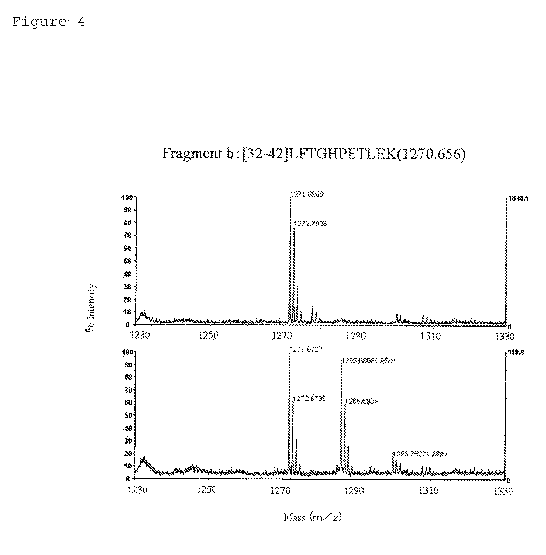

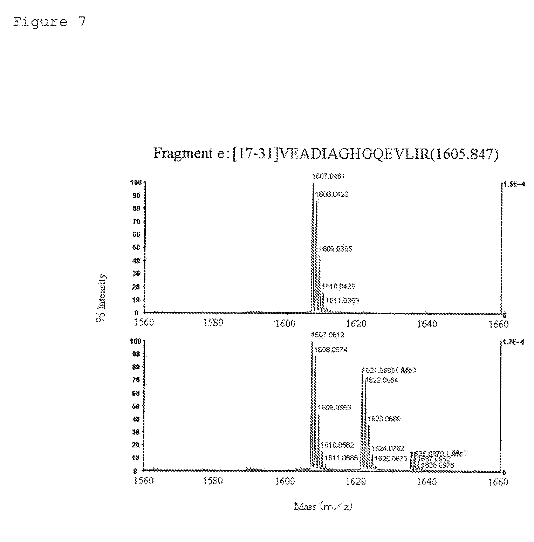

In addition, the same way of containing one glutamic acid residue can apply, even in the case of containing a plurality of the glutamic acid residues in the subject peptide fragment. This is exemplary shown in, for example. Figures 4 and 7. In Figure 4, new peaks around 1285 and 1299 are appeared in comparison between the upper spectrum and lower spectrum. This reflects that two glutamic acid residues are contained in the 32 to 42 residues of the myoglobin. These new peaks appeared at 1285 and 1299 (m/z) are numerals which add multiple number of 14, in view of the theoretical molecular weight of 1270.656 of the Fragment b. The number of 14 is a number which reflects methyl as the substituted residue of methanol used for the reacting step. That is, when a new peak is appeared at a mass greater by the molecular weight of the substituted residue originated from the alcohol used in the reacting step before and after the reacting step with regard to the peak originated from a specific peptides fragment, it can be determined that the specific peptide fragment contains the glutamic acid residue. In addition, when a new peak is appeared at a mass by the molecular weight of the substituted residue and when a new peak is appeared at a mass greater by multiple number of the molecular weight of the substituted residue, it can be determined that the specific peptide fragment contains this multiple number of glutamic acid residues.

On the other hand, in the case that a new peak is not observed before and after the reacting step with regard to the peak originated from the specific peptide fragment, it can be determined that the glutamic acid residue is contained in the specific peptide fragment.

It should be noted that the change of the molecular weight originated from the substituted residue depends on the substituted residue corresponding to the alcohols in the method for modifying a peptide according to the present invention, although the above-mentioned example shows in the case of using methanol as the alcohol for the reacting step (the substituted residue is methyl group). Therefore, the description of the determining step can apply such that it replaced with the molecular weight of the substituted residue used for the reacting step.

These information, that is, number of the glutamic acid residue contained in the specific peptide fragment or an information as to whether there is the glutamic acid residue contained in the specific peptide fragment is extremely useful for examining the assignment of the specific peptide fragment and/or the peptide originated from this peptide fragment, along with the molecular weight as obtained from the mass analysis.

Generally, peptide fragments within the error range of the molecular weight as observed in the measurement are cyclopaedically extracted from the database in the PMF method. That is, peptide fragment(s) which is in the specific range of the molecular weight are cyclopaedically searched, and all these peptide fragments are handled as candidate of the protein. Therefore, the peptide fragment, the candidate protein, which is extracted from the database is based on the molecular weight as one of indexes.

On the other hand, in the method for identifying a peptide according to the present invention, variable number such as presence or absence of the glutamic acid residue or number of the glutamic acid residue is obtained, along with the molecular weight. Therefore, it is possible to utilize the presence or absence of the glutamic acid residue or the number of the glutamic acid residue as the indexes in addition to the molecular weight of the peptide fragment as the above-mentioned indexes for the searching. That is, it is possible to examine based on the number of the glutamic acid residue as the indexes in addition to the cyclopaedical determination of the peptide fragment using the molecular weight as one of indexes in PMF method in the art, thereby being capable of further narrowing down the candidate protein.

### Embodiment

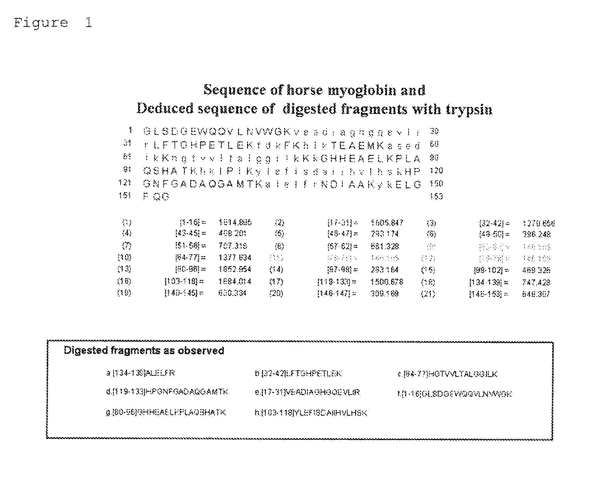

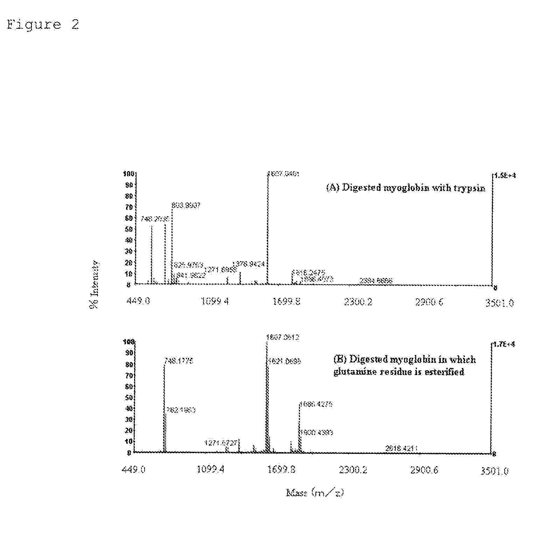

5 µg of myoglobin (Sequence Number 1) was electrophored by SDS-PAGE, followed by staining the band with CBB. The stained band was cut out with 1 mm×1 mm, immersed in a solution of water, methanol and TFA with the ratio of water: methanol : TFA=500: 250: 500 (µL) and stood for 4 hours in room temperature. Then, the gel was washed four times with water for 15 minutes in room temperature. Then, the gel was immersed in a solution of 50 mM ammonium bicarbonate (ABC solution) until the pH in the gel is about. 8 (for example, for about 20 minutes), further dehydrated with 100% acetonitrile solution, and immersed in 12.5 ng/µL trypsin solution for 16 hours in 37°C to perform in-gel digestion. The digested fragment as obtained was analyzed with MALDI-TOF-MS. In addition, digested fragments of non-digested myoglobin with trypsin were similarly analyzed with MALDI-TOF-MS as a control. These results are correctively shown in Figure 1, and the detailed results are shown in Figures 2 to 9.

Figure 1 shows sequence of horse myoglobin, deduced sequence of digested fragment as digested with trypsin and observed sequence of digested fragment. In Figure 1, the upper portion indicates the amino acid sequence of horse myoglobin (Sequence Number 1), the middle portion indicates the deduced trypsin-digested fragment from the amino acid sequence, and the lower portion indicates the digested fragment as observed by MALDI-TOF-MS analysis. Each digested fragment as indicated in the lower portion correspond to 134 to 139 residues (Fragment a; Sequence Number 2), 32 to 42 residues (Fragment b; Sequence Number 3), 64 to 77 residues (Fragment c; Sequence Number 4), 119 to 133 residues (Fragment d; Sequence Number 5), 17 to 31 resides (Fragment e; Sequence Number 6), 1 to 16 residues (Fragment f; Sequence Number 7), 80 to 96 residues (Fragment g; Sequence Number 8) and 103 to 118 residues (Fragment h; Sequence Number 9) of myoglobin, respectively.

Figure 2 is figures showing spectrum of mass analysis of myoglobin as obtained in accordance with the method of the present invention in which the glutamic acid residue thereof is esterified, wherein (A) corresponds to the trypsin digestion of horse myoglobin, and (B) corresponds to the trypsin digestion of horse myoglobin which glutamic acid residue is esterified.

Further, Figures 3 to 9 are that the horizontal axis of the spectrum of mass analysis as indicated in Figure 2 is enlarged and these figures indicates each of Fragments a to h, respectively. Figures 3 to 9 corresponds respectively to:
Figure 3: 134 to 139 residues (Fragment a; Sequence Number 2);
Figure 4 : 32 to 42 residues (Fragment b; Sequence Number 3);

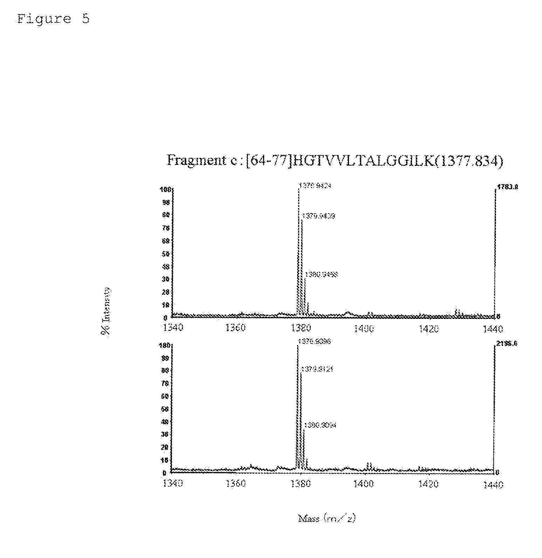

Figure 5 : 64 to 77 residues (Fragment c; Sequence Number 4);

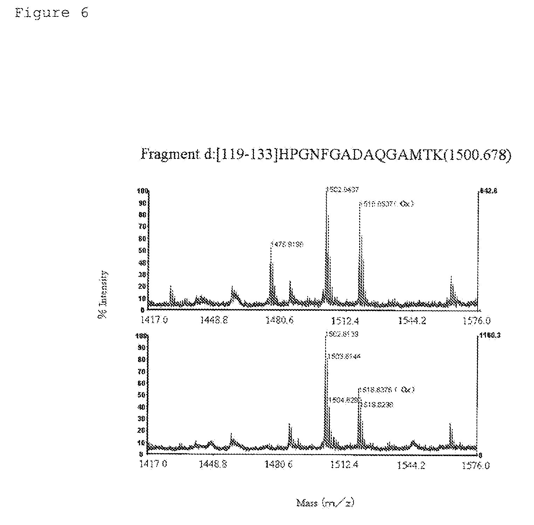

Figure 6: 119 to 133 residues (Fragment d; Sequence Number 5);
Figure 7 : 17 to 31 residues (Fragment e; (Sequence Number 6);

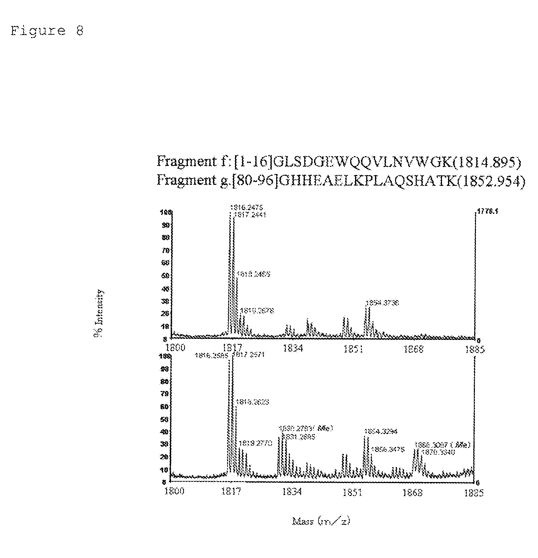

Figure 8 : 1 to 16 residues (Fragment f; Sequence Number 7) and 80 to 96 residues (Fragment g; Sequence Number 8); and
Figure 9 : 103 to 118 residues (Fragment h; Sequence Number 9); of myoglobin. The upper spectrums of Figures 3 to 9 are obtained that the reacting step of the present invention is not performed, and the lower spectrums thereof are obtained that the reacting step of the present invention is not performed. In addition, in Figures 3 to 9, the peak in which "Me" or "Ox" is indicated, following the molecular weight, is a peak originated from the methylated peptide fragment and the oxidized peptide fragment as indicated in each Figure.

According to the comparison between the upper and lower spectrum, in the spectrum of mass analysis of the Fragments a, b, e, and h which contain the glutamic acid residue ("E" as indicated by one character) in the Fragment, among the peaks which are not observed in the upper spectrum but in the lower spectrum, a peak which is shifted from the original peak by 14 of the molecular weight: was observed ("Me" as indicated in each Figure). The shift of the molecular weight is originated from the methyl group of the methanol used for the reacting step of the present invention. Especially, with regard to the Fragments b and e containing the plurality of the glutamic acid residue in the Fragments (Figures 4 and 7), the peak was newly appeared at a position which is shifted frog the original one by 14 of the molecular weight, and the new peak was also appeared at a position which is further shifted from the peak by 14 of the molecular weight (in Figure 4, corresponding respectively to the peaks at 1285.6866 and 1299.7527 with regard to the original peak at 1271.6727, and in Figure 7, corresponding respectively to the peaks at 1621.0698 and 1635.0870 with regard to the original peak at 1607.0612).

It should be noted that, with regard to the peak of the Fragment g (Sequence Number 8) corresponding to 80 to 96 residues of the myoglobin, it is speculated that there was below the detection limit, since, the peak is very slightly detect even in the control and it is considered as a peptide fragment which is difficult to be observed in the spectrum.

On the other hand, with regard to the Fragments c and d (Figures 5 and 6) not containing the glutamic acid residue, even in comparison with the spectrum as obtained from the sample which is or not performed the reacting step of the present invention, the above-mentioned shift of the peak was not observed. It should be noted that two brig peaks of the Fragment d as observed in Figure 6 (in the upper spectrum, 1502.8437 and 1518.8537, and in the lower spectrum, 1502.8139 and 1518.8376) are originated from the oxidation of methionine residue, the difference is different, from the molecular weight of the methyl group.

In addition, for the above-mentioned Fragments, in the method for modifying a peptide according to the present invention, it can be recognized that the modification reaction such as the esterification is not occurred or is less occurred if occurring, in the aspartic residue container in the peptide, and that the modification reaction is specific to the glutamic acid residue. In addition, the identification is possible for the methyl ester of the glutamic acid residue in view of the intensity, even if the esterification of the aspartic residue is occurred.

In addition, in the method for modifying a peptide according to the present invention, due to the use of the perhalogenated carboxylic acid, the spectrum originated from molecule(s) such as Ser-N, Thr-N and Asp-C wherein the molecule has a weak bonding to the strong acid such as hydrochloric acid was not observed, and deamination of glutamine and asparagine is not observed. Therefore, it can be recognized that the method for modifying a peptide according to the present invention is very useful for the structural analysis of protein.

The present invention has been particularly described with reference to preferred aspect of embodiments thereof. While the present invention has described to show the specific embodiments, it is obvious that various modifications and changes is made to these specific embodiments without departing from the broad spirit and scope, of the present invention as defined by the claims. That is, it should not be interpreted that the present invention is not limited to the detailed explanation of the specific embodiments and the accompanied drawing.

## Claims

1. A method for modifying a peptide, wherein the peptide as supported in a substrate and an aqueous solution of perhalogenated carboxylic acid containing an alcohol is reacted to selectively esterify a glutamic acid residue of said peptide.

2. The method for modifying a peptide according to claim 1, wherein carbon number of said alcohol is in the range of 1 to 5.

3. The method for modifying a peptide according to claim 1 or 2, wherein said perhalogenated carboxylic acid is selected from the group consisting of trichloroacetic acid, trifluoroacetic acid, pentafluoropropionic acid and heptafluorobutylic acid.

4. The method for modifying a peptide according to any one of claims 1 to 3, wherein said substrate is polyacrylamide gel.

5. The method for modifying a peptide according to any one of claim 1 to 4, wherein said alcohols is methanol.

6. The method for modifying a peptide according to any one of claims 1 to 5, wherein said perhalogenated carboxylic acid is trifluoroacetic acid.

7. A method for identifying a peptide comprising the steps of:
reacting the peptide as supported in a substrate and an aqueous solution of perhalogenated carboxylic acid containing an alcohol to selectively esterify glutamic acid residue of said peptide;
immersing said substrate in a protease solution to obtain a peptide fragment originated from said peptide;
measuring a molecular weight of said peptide fragment; and
determining said peptide based on said molecular weight.

8. The method for identifying a peptide according to claim 7, wherein said step of determining said peptide based on said molecular weight is that number of glutamic acid residue contained in said peptide and the molecular weight of said peptide simultaneously determine based on a difference between a molecular weight of a peptide fragment originated from the peptide containing the esterified glutamic acid residue and a molecular weight corresponding to said peptide fragment and originated from said peptide.
